(19) Europäisches Patentamt
European Patent Office
Office européen des brevets

(11) **EP 4 129 844 A1**

(12) **EUROPEAN PATENT APPLICATION**
published in accordance with Art. 153(4) EPC

(43) Date of publication:
**08.02.2023 Bulletin 2023/06**

(21) Application number: **21778932.0**

(22) Date of filing: **23.03.2021**

(51) International Patent Classification (IPC):
**B65D 23/08** (1968.09)    **A61J 3/00** (1968.09)
**G01N 1/10** (1968.09)    **G01N 33/48** (1980.01)

(52) Cooperative Patent Classification (CPC):
**A61J 3/00; B65D 23/08; G01N 1/10; G01N 33/48**

(86) International application number:
**PCT/JP2021/011919**

(87) International publication number:
**WO 2021/200385 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2020 JP 2020062014**
**27.10.2020 JP 2020179842**

(71) Applicants:
• **Sekisui Medical Co., Ltd.**
**Tokyo 103-0027 (JP)**
• **Tokuyama Sekisui Co., Ltd.**
**Osaka-shi, Osaka 530-8565 (JP)**

(72) Inventors:
• **UCHIYAMA, Takaya**
**Shunan-shi, Yamaguchi 746-0006 (JP)**
• **KOMAI, Kuniya**
**Shunan-shi, Yamaguchi 746-0006 (JP)**
• **NIUNOYA, Masatoshi**
**Shunan-shi, Yamaguchi 746-0006 (JP)**
• **GOTOU, Yuuki**
**Shunan-shi, Yamaguchi 746-0006 (JP)**
• **ISOGAWA, Hironobu**
**Shunan-shi, Yamaguchi 746-0006 (JP)**

(74) Representative: **Ter Meer Steinmeister & Partner
Patentanwälte mbB
Nymphenburger Straße 4
80335 München (DE)**

(54) **SPECIMEN COLLECTION CONTAINER**

(57) A specimen collection container is provided, the specimen collection container being capable of suppressing a change in a mixing ratio of a specimen collected to a liquid contained even when a long period of time has elapsed after manufacturing. A specimen collection container according to the present invention is a specimen collection container into which a specimen is collected, the specimen collection container including: a container main body having an opening; a plug attached to the opening; a barrier film disposed on an outer surface of the container main body; and a liquid contained in the container main body, the barrier film having a water vapor transmission rate at 40°C and 90% RH of 0.8 g/(m$^2$ · day) or less.

[FIG. 1.]

**Description**

**TECHNICAL FIELD**

The present invention relates to a specimen collection container.

**BACKGROUND ART**

In a clinical examination, a specimen collection container in which the inside of a blood collection tube or the like is decompressed has been widely used (For example, Patent Document 1). The specimen collection container is designed so that a specified amount of a specimen is collected by decompressing the inside.

**Related Art Document**

**Patent Document**

Patent Document 1: JP 2010-154909 A

**SUMMARY OF THE INVENTION**

**PROBLEMS TO BE SOLVED BY THE INVENTION**

A specimen collection container in which the inside is decompressed and a liquid for mixing with a specimen is contained in advance may be used. The specimen collection container is designed so that a collected specimen and a contained liquid are mixed at a predetermined mixing ratio. Examples of the specimen collection container include a vacuum blood collection tube containing a liquid including an anticoagulant.

However, after manufacturing the specimen collection container, the internal pressure of the specimen collection container gradually increases, and the contained liquid gradually evaporates. For this reason, in the conventional specimen collection container, the mixing ratio of the collected specimen to the contained liquid may be changed as time elapses after manufacturing. Note that, although the change in the mixing ratio can be suppressed to some extent by packing the specimen collection container in an aluminum gusset bag, the effect is not sufficient. Further, this method has a problem with an increase in waste.

An object of the present invention is to provide a specimen collection container capable of suppressing a change in a mixing ratio of a collected specimen to a contained liquid even when a long period of time has elapsed after manufacturing.

**MEANS FOR SOLVING THE PROBLEMS**

According to a broad aspect of the present invention, there is provided a specimen collection container into which a specimen is collected, the specimen collection container including: a container main body having an opening; a plug attached to the opening; a barrier film disposed on an outer surface of the container main body; and a liquid contained in the container main body, the barrier film having a water vapor transmission rate at 40°C and 90% RH of 0.8 $g/(m^2 \cdot day)$ or less.

In a specific aspect of a specimen collection container according to the present invention, an air transmission rate of the specimen collection container at 40°C and 0% RH is 0.5 $cc/(m^2 \cdot day \cdot atm)$ or less.

In a specific aspect of a specimen collection container according to the present invention, a water vapor transmission rate of the specimen collection container at 40°C and 0% RH is 1.1 $g/(m^2 \cdot day)$ or less.

In a specific aspect of a specimen collection container according to the present invention, the barrier film is disposed at 0.2 turns or more and 3 turns or less in a circumferential direction of the outer surface of the container main body.

In a specific aspect of a specimen collection container according to the present invention, a surface area of a portion where the barrier film is disposed is 15% or more and 90% or less in 100% of a total surface area of the outer surface of the container main body.

In a specific aspect of a specimen collection container according to the present invention, a thickness of the barrier film before being disposed on the outer surface of the container main body is 5 um or more and 300 um or less.

In a specific aspect of a specimen collection container according to the present invention, the barrier film includes a first film, a first adhesive layer, a second film, and a second adhesive layer in this order, and the second adhesive layer is disposed on the outer surface of the container main body.

In a specific aspect of a specimen collection container according to the present invention, the first film is a barrier film main body, and the second film is a polyethylene terephthalate film.

In a specific aspect of a specimen collection container according to the present invention, the material of the container main body is polyethylene terephthalate or polyethylene naphthalate.

In a specific aspect of a specimen collection container according to the present invention, the specimen is blood, and the liquid is a liquid including an anticoagulant.

In a specific aspect of a specimen collection container according to the present invention, the anticoagulant is citric acid, EDTA, or heparin.

In a specific aspect of a specimen collection container according to the present invention, the amount of the liquid contained in the container main body is 0.1 mL or more and 5 mL or less.

## EFFECT OF THE INVENTION

A specimen collection container according to the present invention is a specimen collection container into which a specimen is collected, the specimen collection container including: a container main body having an opening; a plug attached to the opening; a barrier film disposed on an outer surface of the container main body; and a liquid contained in the container main body, the barrier film having a water vapor transmission rate at 40°C and 90% RH of 0.8 g/(m$^2 \cdot$ day) or less. Since the specimen collection container according to the present invention is provided with the above-described configuration, a change in a mixing ratio of a collected specimen to a contained liquid can be suppressed even when a long period of time has elapsed after manufacturing.

## BRIEF DESCRIPTION OF DRAWINGS

[Fig. 1] Fig.1 is a front cross-sectional view schematically illustrating a specimen collection container according to a first embodiment of the present invention.
[Fig. 2] Fig. 2 is a front cross-sectional view schematically illustrating a specimen collection container according to a second embodiment of the present invention.

## MODES FOR CARRYING OUT THE INVENTION

Hereinafter, the present invention will be described in detail.

A specimen collection container according to the present invention is a specimen collection container into which a specimen is collected, the specimen collection container including: a container main body having an opening; a plug attached to the opening; a barrier film disposed on an outer surface of the container main body; and a liquid contained in the container main body, the barrier film having a water vapor transmission rate at 40°C and 90% RH of 0.8 g/(m$^2 \cdot$ day) or less.

Since the specimen collection container according to the present invention is provided with the above-described configuration, a change in a mixing ratio of a collected specimen to a contained liquid can be suppressed even when a long period of time has elapsed after manufacturing. In the present invention, a change in a mixing ratio of a specimen to a liquid can be suppressed when a specimen is collected using the specimen collection container in which a long period of time has elapsed after manufacturing, compared to a mixing ratio of a specimen to a liquid when a specimen is collected using the specimen collection container in which not much time has elapsed after manufacturing.

In the specimen collection container in which the inside is decompressed and the liquid is contained in advance, the internal pressure gradually increases after manufacturing, and the contained liquid gradually evaporates. In the conventional specimen collection container, the rate of increase in the internal pressure and the rate of evaporation of the liquid are not controlled at all. When the internal pressure in the specimen collection container increases, the amount of the specimen collected decreases. Therefore, in the conventional specimen collection container, even if the specimen and the liquid can be mixed at a prescribed mixing ratio when the specimen is collected using the specimen collection container in which not much time has elapsed after manufacturing, the mixing ratio may be changed when the specimen is collected using the specimen collection container in which a long period of time has elapsed from manufacturing. When the mixing ratio is changed, the inspection accuracy may decrease in an inspection using the specimen.

On the other hand, in the specimen collection container according to the present invention, since the specific barrier film is disposed on the outer surface of the container main body, the relationship between the rate of decrease in the amount of the specimen collected and the rate of evaporation of the contained liquid is likely to be kept constant even when the specimen collection container after a long period of time has elapsed, is used. Therefore, a change in the mixing ratio of the collected specimen to the contained liquid can be suppressed.

A water vapor transmission rate of the specimen collection container at 40°C and 0% RH is preferably 1.1 g/(m$^2$ ▪ day) or less, more preferably 0.9 g/(m$^2$ ▪ day) or less, and still more preferably 0.6 g/(m$^2$ ▪ day) or less. When the water vapor transmission rate is the aforementioned upper limit or less, the effect of the present invention can be further effectively exhibited. Note that the water vapor transmission rate of the specimen collection container at 40°C and 0% RH may be 0.2 g/ (m$^2$ ▪ day) or more and or 0.4 g/ (m$^2$ ▪ day) or more.

The water vapor transmission rate of the specimen collection container at 40°C and 0% RH is measured as follows.

Water is contained in the container main body in which no liquid is contained inside, and the container main body is sealed with the plug to obtain the specimen collection container (specimen collection container in which the inside is not decompressed and water is contained inside) in which the barrier film is disposed on the outer surface of the container main body. The obtained specimen collection container is stored at 40°C and 0% RH. The water vapor transmission rate is calculated using the amount of attenuation of the weight of the specimen collection container before and after storage as a transpiration amount of water. The combination of the container main body, the plug, and the barrier film is preferably a combination satisfying a preferable range of the water vapor transmission rate.

The air transmission rate of the specimen collection container at 40°C and 0% RH is preferably 0.5 cc/ (m$^2$ ▪ day ▪ atm) or less, more preferably 0.4 cc/ (m$^2$ ▪ day ▪ atm) or less, and still more preferably 0.3 cc/ (m$^2$ ▪ day ▪ atm) or less. When the air transmission rate is the aforementioned upper limit or less, the effect of the present invention can be further effectively exhibited. Note that the air transmission rate of the specimen collection container at 40°C and 0% RH may be 0.1 cc/ (m$^2$ ▪ day ▪ atm) or more and or 0.2 cc/ (m$^2$ ▪ day ▪ atm) or more.

The air transmission rate of the specimen collection container at 40°C and 0% RH is measured as follows.

The plug is attached to the container main body in a decompressed state to obtain the specimen collection container in which the barrier film is disposed on the outer surface of the container main body and the inside is decompressed. The obtained specimen collection container is stored at 40°C and 0% RH. Water is collected by suction into the specimen collection container, and the amount of increase in the internal pressure caused due to storage is obtained from the calibration curve of the collecting amount and the internal pressure, and the air transmission rate is calculated.

Hereinafter, members configuring the specimen collection container and the like will be described in detail.

(Barrier film)

The barrier film is disposed on an outer surface of the container main body. The barrier film is preferably stuck on the outer surface of the container main body, and is preferably rolled thereon. The barrier film is preferably a gas barrier film. The barrier film is preferably transparent. In the specimen collection container, when the specimen is collected, the collected specimen is preferably visually recognizable through the barrier film. The barrier film is preferably a transparent barrier film, and is preferably a transparent gas barrier film.

From the viewpoint of exhibiting the effect of the present invention, the water vapor transmission rate of the barrier film at 40°C and 90% RH is 0.8 g/ (m$^2$ ▪ day) or less. The water vapor transmission rate of the barrier film at 40°C and 90% RH is preferably 0.6 g/ (m$^2$ ▪ day) or less, more preferably 0.3 g/ (m$^2$ ▪ day) or less, still more preferably 0.1 g/(m$^2$ ▪ day) or less, and particularly preferably 0.05 g/ (m$^2$ ▪ day) or less. When the water vapor transmission rate is the aforementioned upper limit or less, the effect of the present invention can be further effectively exhibited.

The water vapor transmission rate of the barrier film at 40°C and 90% RH is measured in accordance with JIS K7129 Method B.

The thickness of the barrier film (the thickness of the barrier film before being disposed on the outer surface of the container main body) is preferably 5 um or more, more preferably 30 um or more, and preferably 300 um or less, more preferably 200 um or less. The thickness of the barrier film is a thickness of the barrier film itself. When the thickness of the barrier film is the aforementioned lower limit or more and the aforementioned upper limit or less, the flexibility of the barrier film can be enhanced, and the barrier film can be favorably disposed on the outer surface of the container main body. Therefore, the risk of remaining air bubbles between the container main body and the barrier film can be reduced.

The barrier film is preferably disposed at 0.2 turns or more, more preferably 0.5 turns or more, and preferably 3 turns or less, more preferably 1.5 turns or less in a circumferential direction of the outer surface of the container main body. In this case, the effect of the present invention can be further effectively exhibited.

In 100% of a total surface area of the outer surface of the container main body, the surface area of the portion where the barrier film is disposed is preferably 15% or more, more preferably 30% or more, further preferably 50% or more and particularly preferably 60% or more, and preferably 100% or less, more preferably 95% or less, further preferably 90% or less and particularly preferably 80% or less. When the surface area of the portion where the barrier film is disposed is the aforementioned lower limit or more and the aforementioned upper limit or less, the effect of the present invention can be further effectively exhibited.

The barrier film is preferably a barrier film including a base film and an inorganic oxide layer and more preferably a barrier film including a base film, an inorganic oxide layer, and a barrier coat layer in this order. The base film and the inorganic oxide layer may be directly laminated, or may be laminated with another layer interposed therebetween. The inorganic oxide layer and the barrier coat layer may be directly laminated, or may be laminated with another layer interposed therebetween. Note that the base film, the inorganic oxide layer, and the barrier coat layer will be described later.

The barrier film may or may not have an adhesive layer. From the viewpoint of favorably disposing the barrier film on the outer surface of the container main body, the barrier film preferably has a barrier film main body and an adhesive layer. In the specimen collection container, it is preferable that the adhesive layer is disposed on the outer surface of the container main body and the barrier film main body is disposed outside the adhesive layer. In this case, in the specimen collection container, the barrier film main body may be disposed on the outer surface of the adhesive layer.

The barrier film is preferably a laminated film having a first film that is a barrier film main body and a second film.

From the viewpoint of favorably producing the specimen collection container, the barrier film more preferably has a first film, a first adhesive layer, a second film, and a second adhesive layer in this order. In this case, the outer surface of the first film is preferably the outer surface of the barrier film. In addition, in this case, the second adhesive layer is preferably disposed on the outer surface of the container main body. In addition, in this case, the first film may be a barrier film main body, and the second film may be a barrier film main body.

The first film and the first adhesive layer may be directly laminated, or may be laminated with another layer interposed therebetween. The first adhesive layer and the second film may be directly laminated, or may be laminated with another layer interposed therebetween. The second film and the second adhesive layer may be directly laminated, or may be laminated with another layer interposed therebetween.

From the viewpoint of reducing a thickness of the barrier film, the first film and the first adhesive layer are preferably directly laminated, the first adhesive layer and the second film are preferably directly laminated, and the second film and the second adhesive layer are preferably directly laminated.

Hereinafter, layers configuring a barrier film will be further described.

<Barrier film main body, first and second films>

The barrier film preferably has a barrier film main body. Note that the barrier film may be only the barrier film main body.

The water vapor transmission rate of the barrier film main body at 40°C and 90% RH is preferably 0.8 g/(m$^2$ ▪ day) or less, more preferably 0.6 g/(m$^2$ ▪ day) or less, still more preferably 0.3 g/ (m$^2$ ▪ day) or less, particularly preferably 0.1 g/ (m$^2$ ▪ day) or less, and most preferably 0.05 g/(m$^2$ ▪ day) or less.

The water vapor transmission rate of the barrier film main body at 40°C and 90% RH is measured in accordance with JIS K7129 Method B.

The barrier film main body preferably includes a base film and an inorganic oxide layer and more preferably includes a base film, an inorganic oxide layer, and a barrier coat layer in this order. The base film and the inorganic oxide layer may be directly laminated, or may be laminated with another layer interposed therebetween. The inorganic oxide layer and the barrier coat layer may be directly laminated, or may be laminated with another layer interposed therebetween.

Examples of the base film include a polyethylene terephthalate film, a polyethylene naphthalate film, a nylon film, a polyethylene film, a polypropylene film, a polystyrene film, a polyamide film, a polycarbonate film, a polyacrylonitrile film, and a polyimide film.

The base film is preferably transparent.

The inorganic oxide layer is preferably a layer having a gas barrier property. The inorganic oxide layer is preferably an inorganic oxide vapor-deposited layer in which an inorganic oxide is vapor-deposited on the surface of the base film.

Examples of the inorganic oxide included in the inorganic oxide layer include aluminum oxide, silicon oxide, tin oxide, and magnesium oxide. Only one kind of the inorganic oxide may be used, or two or more kinds thereof may be used in combination.

From the viewpoint of enhancing the gas barrier property, the inorganic oxide is preferably aluminum oxide or silicon oxide.

The barrier coat layer is a layer having a gas barrier property. By having the barrier coat layer, oxidation of the inorganic oxide can be effectively suppressed, and breakage of the inorganic oxide layer due to external impact or the like can be suppressed.

As a material of the barrier coat layer, a conventionally well-known material used as a barrier coat layer can be used. Examples of the material of the barrier coat layer include a composition including a hydrolysis product of alkoxysilane and a water-soluble polymer, polyethylene terephthalate, and nylon. Only one kind of the material of the barrier coat layer may be used, or two or more kinds thereof may be used in combination.

Examples of the water-soluble polymer include a polyvinyl alcohol-based resin and an ethylene-vinyl alcohol copolymer. Only one kind of the water-soluble polymer may be used, or two or more kinds thereof may be used in combination.

Among the base film and the inorganic oxide layer, the base film is preferably disposed on the container main body side. Among the base film and the barrier coat layer, the base film is preferably disposed on the container main body side.

From the viewpoint of lamination processing and quality guarantee of the barrier film, the first film is preferably the barrier film main body. In the specimen collection container, the outer surface of the first film is preferably the outer surface of the barrier film.

Commercially available products can also be used as the barrier film, the barrier film main body, and the first film which is the barrier film main body. Examples of the commercially available product include "GX-P-F" and "GL-AEC-F" manufactured by TOPPAN INC., "IB-PET-PXB2" manufactured by Dai Nippon Printing Co., Ltd., "Barrialox 1011 SBR2" manufactured by TORAY ADVANCED FILM CO., LTD., "V BARRIER" manufactured by Mitsui Chemicals Tohcello, Inc., "TECHBARRIER" manufactured by Mitsubishi Chemical Corporation. In addition, examples of the barrier film include a film including these commercially available films.

The second film may be the barrier film main body, and may not be the barrier film main body. When the first film is the barrier film main body, the second film is preferably not the barrier film main body, and is preferably a resin film.

Examples of the second film include a polyethylene terephthalate film, a polyethylene naphthalate film, a nylon film, a polyethylene film, a polypropylene film, a polystyrene film, a polyamide film, a polycarbonate film, a polyacrylonitrile film, and a polyimide film.

The second film is preferably a polyethylene terephthalate film. In this case, an adhesive can be applied well, and lamination processability can also be enhanced.

The thickness of the second film is preferably 1 um or more, more preferably 10 um or more, and preferably 80 um or less, more preferably 50 um or less. The thickness of the second film is a thickness of the second film itself. When the thickness of the second film is the aforementioned lower limit or more and the aforementioned upper limit or less, the flexibility of the barrier film can be enhanced, and the barrier film can be favorably disposed on the outer surface of the container main body. Therefore, the risk of remaining air bubbles between the container main body and the barrier film can be reduced.

<Adhesive layer (first and second adhesive layers)>

The barrier film preferably has an adhesive layer. The adhesive layer is a layer formed of an adhesive.

The first adhesive layer is disposed between the first film and the second film. The second adhesive layer is disposed between the second film and the outer surface of the container main body. The second adhesive layer is preferably disposed on the outer surface of the container main body.

The material (material of the first and second adhesive layers) of the adhesive layer is not particularly limited, and a conventionally well-known adhesive can be used. Examples of the adhesive include a silicone-based adhesive, a urethane-based adhesive, and an acryl-based adhesive. Only one kind of the adhesive may be used, or two or more kinds thereof may be used in combination.

From the viewpoint of enhancing the adhesive strength, the adhesive is preferably an acrylic-based adhesive or a silicone-based adhesive.

The thickness of the first adhesive layer is preferably 1 um or more, more preferably 10 um or more, and preferably 80 um or less, more preferably 50 um or less. The thickness of the first adhesive layer is a thickness of the first adhesive layer itself. When the thickness of the first adhesive layer is the aforementioned lower limit or more and the aforementioned upper limit or less, the adhesive strength can be enhanced.

The thickness of the second adhesive layer is preferably 1 um or more, more preferably 10 um or more, and preferably 80 um or less, more preferably 50 um or less. The thickness of the second adhesive layer is a thickness of the second adhesive layer itself. When the thickness of the second adhesive layer is the aforementioned lower limit or more and the aforementioned upper limit or less, the adhesive strength can be enhanced.

(Container main body)

The container main body has an opening. The shape of the container main body is not particularly limited. The shape of the container main body is preferably a bottomed container, and more preferably a bottomed tubular container.

The material of the container main body is not particularly limited. Examples of the material of the container main body include a thermoplastic resin such as polyethylene, polypropylene, polystyrene, polyethylene terephthalate, polyethylene naphthalate, polymethyl methacrylate, and polyacrylonitrile; a thermosetting resin such as an unsaturated polyester resin, an epoxy resin, and an epoxy-acrylate resin; a modified natural resin such as cellulose acetate, cellulose propionate, ethyl cellulose, and ethyl chitin; silicate glass such as soda lime glass, phosphosilicate glass, and borosilicate glass, and glass such as quartz glass. Only one kind of these components may be used, or two or more kinds thereof may be used in combination.

From the viewpoint of further effectively exhibiting the effect of the present invention, the material of the container main body is preferably polyethylene terephthalate or polyethylene naphthalate, and more preferably polyethylene terephthalate.

When the material of the container main body is polyethylene terephthalate, the water vapor transmission rate of the barrier film at 40°C and 90% RH is preferably 0.5 g/(m$^2$ ▪ day) or less, more preferably 0.2 g/ (m$^2$ ▪ day) or less, still more preferably 0.1 g/ (m$^2$ ▪ day) or less, and particularly preferably 0.05 g/ (m$^2$ ▪ day) or less.

(Plug)

The plug is attached to the opening of the container main body. As the plug, a conventionally well-known plug can be used. The plug is preferably a plug having a material and a shape that can be airtightly and liquid-tightly attached to the opening of the container main body. From the viewpoint of easily collecting the specimen in the specimen collection container, the plug is preferably configured such that a needle can be punctured therethrough.

Examples of the plug include a plug having a shape fitted to the opening of the container main body and a sheet-like seal plug.

In addition, the plug may be a plug including a plug main body such as a rubber plug and a cap member configured plastic or the like. In this case, when the plug is pulled out from the opening of the container main body after collecting a body fluid such as blood, the risk that the body fluid comes into contact with a human body can be suppressed.

Examples of the material of the plug (or the plug main body) include a synthetic resin, an elastomer, a rubber, and a metal foil. Examples of the rubber include a butyl rubber and a halogenated butyl rubber. Examples of the metal foil include an aluminum foil. From the viewpoint of enhancing the sealing property, the material of the plug (or the plug main body) is preferably a butyl rubber or a halogenated butyl rubber. The plug (or the plug main body) is preferably a butyl rubber plug or a halogenated butyl rubber plug.

(Specimen and liquid)

The type and amount of a liquid contained in the container main body and the type and amount of a specimen collected are not particularly limited.

Examples of the specimen include a living body-originated sample. Specific examples of the specimen include blood, plasma, urine, and cerebrospinal fluid.

When the specimen is blood, the specimen collection container is a blood collection container. The specimen collection container is preferably a blood collection tube, and more preferably a vacuum blood collection tube.

The solvent of the liquid is preferably water.

When the specimen is blood, the liquid is preferably a liquid including an anticoagulant, and more preferably a liquid including an anticoagulant and water.

Examples of the anticoagulant include citric acid, oxalic acid, EDTA (ethylenediaminetetraacetic acid), and heparin. Only one kind of the anticoagulant may be used, or two or more kinds thereof may be used in combination.

The anticoagulant preferably contains citric acid, EDTA, or heparin, and is more preferably citric acid, EDTA, or heparin. When the specimen is blood, the liquid is preferably a liquid including at least one of citric acid, EDTA, and heparin.

The amount of the liquid contained in the container main body may be 0.1 mL or more and 5 mL or less.

(Other details of specimen collection container)

The inside of the specimen collection container is decompressed. The internal pressure of the specimen collection container is appropriately changed according to the size of the specimen collection container and the amount of the specimen collected.

The amount of the specimen collected in the specimen collection container is not particularly limited. The amount of the specimen collected in the specimen collection container may be 0.5 mL or more, 4.5 mL or more, or 7.6 mL or more. The amount of the specimen collected in the specimen collection container may be 10 mL or less, 5.5 mL or less, or 2.0 mL or less.

Hereinafter, specific embodiments of the present invention will be described with reference to the drawings. Note that, in the following drawings, the size, thickness, shape, and the like may be different from the actual size, thickness, shape, and the like for convenience of illustration.

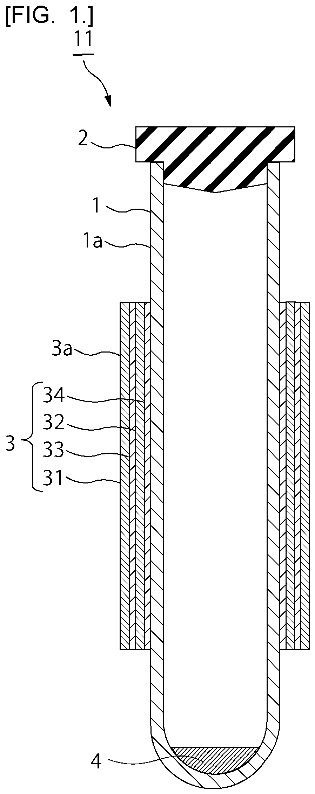

Fig. 1 is a front cross-sectional view schematically showing a specimen collection container according to a first embodiment of the present invention.

A specimen collection container 11 shown in Fig. 1 includes a container main body 1, a plug 2, a barrier film 3, and a liquid 4. The container main body 1 is a tubular container. The container main body 1 has an opening at one end and a closed bottom at the other end. The plug 2 is attached to an opening of the container main body 1. The barrier film 3 is disposed in a circumferential direction of an outer surface 1a of the container main body 1. The barrier film 3 is disposed on a part of the outer surface 1a of the container main body 1. The barrier film 3 is rolled on the outer surface 1a of the container main body 1. The liquid 4 is contained in the container main body 1.

The barrier film 3 includes a first film 31, a first adhesive layer 33, a second film 32, and a second adhesive layer 34 in this order from the outside toward the inside of the specimen collection container 11. The second adhesive layer 34 is disposed on the outer surface 1a of the container main body 1. The second film 32 is disposed on the surface of the second adhesive layer 34 on the opposite side to the container main body 1. The first adhesive layer 33 is disposed on the surface of the second film 32 on the opposite side to the second adhesive layer 34. The first film 31 is disposed on a surface of the first adhesive layer 33 on the opposite side to the second film 32.

In the barrier film 3, the first film 31 is the barrier film main body, and the second film 32 is a polyethylene terephthalate film.

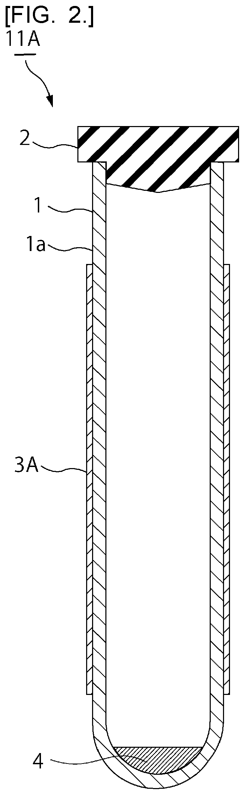

Fig. 2 is a front cross-sectional view schematically showing a specimen collection container according to a second embodiment of the present invention.

A specimen collection container 11A shown in Fig. 2 includes a container main body 1, a plug 2, a barrier film 3A, and a liquid 4. The inside of a specimen collection container 11A is decompressed. The container main body 1 is a tubular container main body. The container main body 1 has an opening at one end and a closed bottom at the other end. The plug 2 is attached to an opening of the container main body 1. The barrier film 3A is disposed in a circumferential direction of an outer surface 1a of the container main body 1. The barrier film 3A is disposed on a part of the outer surface 1a of the container main body 1. The barrier film 3A is rolled on the outer surface 1a of the container main body 1. The liquid 4 is contained in the container main body 1.

The specimen collection container may include other components other than the liquid in the container main body. Examples of the other components include a serum or plasma separation agent, a serum or plasma separation jig, a formaldehyde releasing substance, dextran, ficoll, and magnetic beads.

From the viewpoint of preventing bacterial infection, the inside of the specimen collection container is preferably sterilized in accordance with the standards described in ISO and JIS.

Hereinafter, the present invention will be specifically described with reference to Examples and Comparative Examples. The present invention is not limited to the following Examples.

(Container main body)

The following container main bodies A, B, and C having the shapes shown in Fig. 1 were prepared.

Container main body A:

Length 75 mm × outer diameter 13 mm (length: distance between one end (opening end) and the other end)
Material: polyethylene terephthalate (PET)

Container main body B:

Length 100 mm × outer diameter 16 mm (length: distance between one end (opening end) and the other end)
Material: polyethylene terephthalate (PET)

Container main body C:

Length 75 mm × outer diameter 13 mm (length: distance between one end (opening end) and the other end)
Material: polyethylene naphthalate (PEN)

(Plug)

A rubber plug (butyl rubber plug) having the shape shown in Fig. 1 and attachable to the opening of the container main body was prepared.

(Barrier film)

Barrier film A (a laminated film having "GX-P-F" (thickness of 12 um) manufactured by TOPPAN INC. and a polyethylene terephthalate film (PET film, thickness of 25 um) (laminated product))

Barrier film B ("Transparent 50-SN" manufactured by NEION Film Coatings Corp., a polyethylene terephthalate film (transparent PET film), thickness: of 50 um)

Barrier film C (a laminated film having an ethylene-vinyl alcohol copolymer (EVOH) film (thickness of 12 um) that is "E105B" manufactured by KURARAY CO., LTD. and a polyethylene terephthalate film (Transparent PET film, thickness of 25 um) (laminated product))

Barrier film D (produced by the following method)

Barrier film E ("IB-PET-PXB2" manufactured by Dai Nippon Printing Co., Ltd., thickness of 22 um)

Production of barrier film D:

The following materials were prepared.

Barrier film main body: "GX-P-F" (thickness of 12 um) manufactured by TOPPAN INC.

Polyethylene terephthalate film (PET film) (thickness of 25 $\mu$m)

Adhesive

Release paper

The barrier film main body, the adhesive, the PET film, the adhesive and the release paper were laminated in this order and subjected to lamination processing to obtain a laminated film (laminated film having the barrier film and the release paper) having a layer structure of the barrier film main body (thickness of 12 um)/the first adhesive layer/the PET film (thickness of 25 um)/the second adhesive layer/the release paper. The obtained barrier film D is a laminated film (laminated product) having the barrier film main body (first film), the first adhesive layer, the PET film (second film), and the second adhesive layer in this order, and having a thickness of 66 um.

The water vapor transmission rates of the barrier films A to E at 40°C and 90% RH are shown in Table 1 below. Note that this water vapor transmission rate is a value measured in accordance with JIS K7129 Method B.

[Table 1]

|  | Water vapor transmission rate (g/ (m$^2$ ▪ day)) at 40°C and 90% RH |
|---|---|
| Barrier film A | 0.05 |
| Barrier film B | 1.8 |
| Barrier film C | 5.8 |
| Barrier film D | 0.05 |
| Barrier film E | 0.1 |

(Liquid)

Aqueous solution of 3.2 w/v% citric acid

ACD-A solution (manufactured by Terumo Corporation: 2.2 w/v% sodium citrate hydrate, 0.80 w/v% citric acid hydrate, 2.20 w/v% glucose)

(Example 1)

Manufacture of specimen collection container:
A barrier film A having a length of 50 mm and a width of 40 mm was prepared. The barrier film A was rolled one turn on the outer circumferential surface of the container main body A such that the longitudinal direction of the barrier film A corresponded to the length direction of the container main body A. In addition, about 0.2 mL of an aqueous solution of 3.2 w/v% citric acid was added into the container main body A. Subsequently, the pressure was reduced to 57.8 kPa, and the specimen collection container (blood collection tube) was manufactured by sealing with the plug.

(Example 2)

A specimen collection container in which the barrier film A was rolled 0.5 turns on the outer circumferential surface of the container main body A was manufactured in the same manner as in Example 1 except that the barrier film A having a length of 50 mm and a width of 20 mm was used.

(Example 3)

A barrier film A having a length of 75 mm and a width of 48 mm was prepared. The barrier film A was rolled one turn on the outer circumferential surface of the container main body B such that the longitudinal direction of the barrier film A corresponded to the length direction of the container main body B. In addition, about 1.5 mL of the ACD-A solution was added into the container main body B. Subsequently, the pressure was reduced to 19.9 kPa, and the specimen collection container (blood collection tube) was manufactured by sealing with the plug.

(Example 4)

A specimen collection container in which the barrier film A was rolled 0.5 turns on the outer circumferential surface of the container main body B was manufactured in the same manner as in Example 3 except that the barrier film A having a length of 75 mm and a width of 24 mm was used.

(Example 5)

The laminated film having the barrier film D and the release paper was cut into a size of a length of 50 mm and a width of 40 mm. On the laminated film after cutting, the release paper was peeled off to expose the second adhesive layer. The barrier film D was rolled one turn from the second adhesive layer side on the outer surface of the container main body C such that the longitudinal direction of the barrier film corresponded to the length direction of the container main body C. In addition, 0.20 mL of an aqueous solution of 3.2 w/v% citric acid was added into the container main body C. Subsequently, the pressure was reduced to 57.8 kPa, and the specimen collection container (blood collection tube) was manufactured by sealing with the plug.

(Example 6)

A specimen collection container in which the barrier film D was rolled 0.75 turns on the outer circumferential surface of the container main body C was manufactured in the same manner as in Example 5 except that the laminated film having the barrier film D and the release paper was cut into a size of a length of 50 mm and a width of 30 mm, and the barrier film D obtained from the laminated film after cutting was used.

(Example 7)

The laminated film having the barrier film D and the release paper was cut into a size of a length of 75 mm and a width of 48 mm. On the laminated film after cutting, the release paper was peeled off to expose the second adhesive layer. The barrier film D was rolled one turn from the second adhesive layer side on the outer surface of the container main body B such that the longitudinal direction of the barrier film corresponded to the length direction of the container main body B. In addition, about 1.5 mL of the ACD-A solution was added into the container main body B. Subsequently, the pressure was reduced to 19.9 kPa, and the specimen collection container (blood collection tube) was manufactured by sealing with the plug.

(Example 8)

A specimen collection container in which the barrier film D was rolled 0.5 turns on the outer circumferential surface of the container main body B was manufactured in the same manner as in Example 7 except that the laminated film having the barrier film D and the release paper was cut into a size of a length of 75 mm and a width of 24 mm, and the barrier film D obtained from the laminated film after cutting was used.

(Example 9)

The laminated film having the barrier film D and the release paper was cut into a size of a length of 50 mm and a width of 40 mm. On the laminated film after cutting, the release paper was peeled off to expose the second adhesive layer. The barrier film D was rolled one turn from the second adhesive layer side on the outer surface of the container main body A such that the longitudinal direction of the barrier film corresponded to the length direction of the container main body A. In addition, about 0.2 mL of an aqueous solution of 3.2 w/v% citric acid was added into the container main body A. Subsequently, the pressure was reduced to 57.8 kPa, and the specimen collection container (blood collection tube) was manufactured by sealing with the plug.

(Example 10)

A specimen collection container in which the barrier film D was rolled 0.5 turns on the outer circumferential surface of the container main body A was manufactured in the same manner as in Example 9 except that the laminated film having the barrier film D and the release paper was cut into a size of a length of 50 mm and a width of 20 mm, and the barrier film D obtained from the laminated film after cutting film was used.

(Example 11)

A specimen collection container was manufactured in the same manner as in Example 7 except that the barrier film D was rolled two turns from the second adhesive layer side on the outer surface of the container main body B.

(Example 12)

A specimen collection container was manufactured in the same manner as in Example 7 except that the barrier film D was rolled three turns from the second adhesive layer side on the outer surface of the container main body B.

(Example 13)

A barrier film E having a length of 75 mm and a width of 48 mm was prepared. This barrier film E was rolled one turn on the outer circumferential surface of the container main body B such that the longitudinal direction of the barrier film E corresponded to the length direction of the container main body B. In addition, about 1.5 mL of the ACD-A solution was added into the container main body B. Subsequently, the pressure was reduced to 19.9 kPa, and the specimen collection container (blood collection tube) was manufactured by sealing with the plug.

(Example 14)

A specimen collection container in which the barrier film E was rolled 0.5 turns on the outer circumferential surface of the container main body B was manufactured in the same manner as in Example 13 except that the barrier film E having a length of 75 mm and a width of 24 mm was used.

(Comparative Example 1)

A specimen collection container was manufactured in the same manner as in Example 1 except that no barrier film was used.

(Comparative Example 2)

The specimen collection container obtained in Comparative Example 1, a filter paper (manufactured by ADVANTEC TOYO KAISHA, Ltd.), and an oxygen scavenger (manufactured by MITSUBISHI GAS CHEMICAL COMPANY, INC.) were contained in an aluminum gusset bag (manufactured by Dai Nippon Printing Co., Ltd.), and the aluminum gusset bag was sealed. In this way, the specimen collection container contained in the aluminum gusset bag was manufactured.

(Comparative Example 3)

A specimen collection container in which the barrier film B was rolled one turn on the outer circumferential surface of the container main body A was manufactured in the same manner as in Example 1 except that the barrier film B having a length of 50 mm and a width of 40 mm was used.

(Comparative Example 4)

A specimen collection container in which the barrier film C was rolled one turn on the outer circumferential surface of the container main body A was manufactured in the same manner as in Example 1 except that the barrier film C having a length of 50 mm and a width of 40 mm was used.

(Evaluation)

(1) Air transmission rate of specimen collection container (40°C and 0% RH)

The obtained specimen collection container is stored in a thermo-hygrostat at 40°C and 0% RH. Water was collected by suction into the specimen collection container, and the amount of increase in the internal pressure caused due to storage was obtained from the calibration curve of the collecting amount and the internal pressure, and the air transmission rate was calculated. Note that, in Comparative Example 2, the specimen collection container was stored in a thermo-hygrostat in a state of being contained in the aluminum gusset bag, and the above operation was performed.

(2) Water vapor transmission rate of specimen collection container (40°C and 0% RH)

In the combination of the container main body and the barrier film used in each Example and each Comparative Example, water was contained in the container main body in which no liquid was contained inside, and the container main body was sealed with the plug to obtain a specimen collection container (specimen collection container in which the inside is not decompressed and water is contained inside) in which the barrier film was disposed on the outer surface of the container main body. The obtained specimen collection container is stored in a thermo-hygrostat at 40°C and 0% RH. The water vapor transmission rate is calculated using the amount of attenuation of the weight of the specimen collection container before and after storage as a transpiration amount of water. Note that, in Comparative Example 2, the specimen collection container was stored in a thermo-hygrostat in a state of being contained in the aluminum gusset bag, and the above operation was performed.

(3) Storage of specimen collection container

The obtained specimen collection container is stored in a thermo-hygrostat at 40°C and 0% RH for 70 days. The storage at 40°C for 70 days corresponds to the storage at 25°C for 1 year. Note that, in Comparative Example 2, the specimen collection container was stored in a thermo-hygrostat in a state of being contained in the aluminum gusset bag.

(3-1) Amount of liquid contained

The amounts of liquid contained in the specimen collection container immediately after manufacturing and after storage were measured.

(3-2) Amount of specimen collected

Blood was collected into the specimen collection container immediately after manufacturing and after storage using a blood collection needle. The amount of blood collected was measured.

(3-3) Mixing ratio of liquid to specimen and change rate of mixing ratio

From the amount of the liquid and the amount of the specimen measured in the above (3-1) and (3-2), the mixing ratio of the liquid to the specimen in the specimen collection container immediately after manufacturing and after storage was calculated by the following formula (1). In addition, from the calculated mixing ratio, the change rate of the mixing ratio was calculated by the following formula (2).

Mixing ratio = Amount of specimen (g)/Amount of liquid (mL) (1)

Change rate (%) of mixing ratio = (Y - X)/X × 100 (2)

X: Mixing ratio in specimen collection container immediately after manufacturing
Y: Mixing ratio in specimen collection container after storage

The configurations and results are shown in Tables 2 to 7 below.

[Table 2]

| | | Example 1 | | Example 2 | | Example 3 | |
|---|---|---|---|---|---|---|---|
| Container main body | Type | A | | A | | B | |
| | Material | PET | | PET | | PET | |
| | Length $\times$ outer diameter (mm) | 75 $\times$ 13 | | 75 $\times$ 13 | | 100 $\times$ 16 | |
| Barrier film | Type | A | | A | | A | |
| | Thickness ($\mu$m) | 37 | | 37 | | 37 | |
| | Length $\times$ width (mm) | 50 $\times$ 40 | | 50 $\times$ 20 | | 75 $\times$ 48 | |
| | Surface area (%) of portion where barrier film is disposed in 100% of total surface area of outer surface of container main body | 65 | | 33 | | 72 | |
| | Number of turns (circumference) of barrier film on outer circumferential surface of container main body | 1 | | 0.5 | | 1 | |
| Liquid | Type | Aqueous solution of citric acid | | Aqueous solution of citric acid | | ACD-A solution | |
| Packaging material | | - | | - | | - | |
| Water vapor transmission rate (g/(m$^2$ $\cdot$ day)) at 40°C and 0% RH of specimen collection container | | 0.439 | | 0.825 | | 0.439 | |
| Air transmission rate (cc/ (m$^2$ $\cdot$ day $\cdot$ atm)) at 40°C and 0% RH of specimen collection container | | 0.205 | | 0.484 | | 0.205 | |
| | | Immediately after manufacturing | After storage | Immediately after manufacturing | After storage | Immediately after manufacturing | After storage |
| Amount of liquid contained (mL) | | 0.2 | 0.18 | 0.2 | 0.168 | 1.5 | 1.35 |
| Amount of specimen collected (g) | | 1.80 | 1.75 | 1.80 | 1.64 | 8.50 | 8.25 |
| Mixing ratio of liquid to specimen (amount of specimen/amount of liquid) | | 9.00 | 9.70 | 9.00 | 9.75 | 5.67 | 6.11 |
| Change rate (%) of mixing ratio | | 7.8% | | 8.3% | | 7.8% | |

EP 4 129 844 A1

[Table 3]

| | | | Example 4 | | Example 5 | | Example 6 | |
|---|---|---|---|---|---|---|---|---|
| Container main body | | Type | B | | C | | C | |
| | | Material | PET | | PEN | | PEN | |
| | | Length × outer diameter (mm) | 100 × 16 | | 75 × 13 | | 75 × 13 | |
| Barrier film | | Type | A | | D | | D | |
| | | Thickness (μm) | 37 | | 66 | | 66 | |
| | | Length × width (mm) | 75 × 24 | | 50 × 40 | | 50 × 30 | |
| | | Surface area (%) of portion where barrier film is disposed in 100% of total surface area of outer surface of container main body | 36 | | 65 | | 51 | |
| | | Number of turns (circumference) of barrier film on outer circumferential surface of container main body | 0.5 | | 1 | | 0.75 | |
| Liquid | | Type | ACD-A solution | | Aqueous solution of citric acid | | Aqueous solution of citric acid | |
| Packaging material | | | - | | - | | - | |
| Water vapor transmission rate (g/(m² ▪ day)) at 40°C and 0% RH of specimen collection container | | | 0.825 | | 0.329 | | 0.439 | |
| Air transmission rate (cc/ (m² ▪ day ▪ atm)) at 40°C and 0% RH of specimen collection container | | | 0.484 | | 0.093 | | 0.165 | |
| | | | Immediately after manufacturing | After storage | Immediately after manufacturing | After storage | Immediately after manufacturing | After storage |
| Amount of liquid contained (mL) | | | 1.5 | 1.26 | 0.20 | 0.185 | 0.20 | 0.180 |
| Amount of specimen collected (g) | | | 8.50 | 7.74 | 1.80 | 1.78 | 1.80 | 1.76 |
| Mixing ratio of liquid to specimen (amount of specimen/amount of liquid) | | | 5.67 | 6.14 | 9.00 | 9.61 | 9.00 | 9.78 |
| Change rate (%) of mixing ratio | | | 8.3% | | 6.7% | | 8.6% | |

[Table 4]

| | | Example 7 | | Example 8 | | Example 9 | |
|---|---|---|---|---|---|---|---|
| Container main body | Type | B | | B | | A | |
| | Material | PET | | PET | | PET | |
| | Length × outer diameter (mm) | 100 × 16 | | 100 × 16 | | 75 × 13 | |
| Barrier film | Type | D | | D | | D | |
| | Thickness ($\mu$m) | 66 | | 66 | | 66 | |
| | Length × width (mm) | 75 × 48 | | 75 × 24 | | 50 × 40 | |
| | Surface area (%) of portion where barrier film is disposed in 100% of total surface area of outer surface of container main body | 72 | | 36 | | 65 | |
| | Number of turns (circumference) of barrier film on outer circumferential surface of container main body | 1 | | 0.5 | | 1 | |
| Liquid | Type | ACD-A solution | | ACD-A solution | | Aqueous solution of citric acid | |
| Packaging material | | - | | - | | - | |
| Water vapor transmission rate (g/(m² ▪ day)) at 40°C and 0% RH of specimen collection container | | 0.440 | | 0.830 | | 0.440 | |
| Air transmission rate (cc/ (m² ▪ day ▪ atm)) at 40°C and 0% RH of specimen collection container | | 0.215 | | 0.490 | | 0.215 | |
| | | Immediately after manufacturing | After storage | Immediately after manufacturing | After storage | Immediately after manufacturing | After storage |
| Amount of liquid contained (mL) | | 1.5 | 1.34 | 1.5 | 1.25 | 0.2 | 0.178 |
| Amount of specimen collected (g) | | 8.50 | 8.16 | 8.50 | 7.65 | 1.80 | 1.73 |
| Mixing ratio of liquid to specimen (amount of specimen/amount of liquid) | | 5.67 | 6.11 | 5.67 | 6.14 | 9.00 | 9.71 |
| Change rate (%) of mixing ratio | | 7.9% | | 8.4% | | 7.9% | |

EP 4 129 844 A1

[Table 5]

| | | | Example 10 | | Example 11 | | Example 12 | |
|---|---|---|---|---|---|---|---|---|
| Container main body | | Type | A | | B | | B | |
| | | Material | PET | | PET | | PET | |
| | | Length × outer diameter (mm) | 75 × 13 | | 100 × 16 | | 100 × 16 | |
| Barrier film | | Type | D | | D | | D | |
| | | Thickness (μm) | 66 | | 66 | | 66 | |
| | | Length × width (mm) | 50 × 20 | | 75 × 48 | | 75 × 48 | |
| | | Surface area (%) of portion where barrier film is disposed in 100% of total surface area of outer surface of container main body | 33 | | 72 | | 36 | |
| | | Number of turns (circumference) of barrier film on outer circumferential surface of container main body | 0.5 | | 2 | | 3 | |
| Liquid | | Type | Aqueous solution of citric acid | | ACD-A solution | | ACD-A solution | |
| Packaging material | | | - | | - | | - | |
| Water vapor transmission rate (g/($m^2$ ▪ day)) at 40°C and 0% RH of specimen collection container | | | 0.830 | | 0.359 | | 0.239 | |
| Air transmission rate (cc/ ($m^2$ ▪ day ▪ atm)) at 40°C and 0% RH of specimen collection container | | | 0.490 | | 0.200 | | 0.198 | |
| | | | Immediately after manufacturing | After storage | Immediately after manufacturing | After storage | Immediately after manufacturing | After storage |
| Amount of liquid contained (mL) | | | 0.2 | 0.166 | 1.5 | 1.395 | 1.5 | 1.425 |
| Amount of specimen collected (g) | | | 1.80 | 1.62 | 8.50 | 8.25 | 8.50 | 8.25 |
| Mixing ratio of liquid to specimen (amount of specimen/amount of liquid) | | | 9.00 | 9.76 | 5.67 | 5.91 | 5.67 | 5.79 |
| Change rate (%) of mixing ratio | | | 8.4% | | 4.3% | | 2.1% | |

[Table 6]

| | | | Example 13 | | Example 14 | |
|---|---|---|---|---|---|---|
| Container main body | | Type | B | | B | |
| | | Material | PET | | PET | |
| | | Length × outer diameter (mm) | 100 × 16 | | 100 × 16 | |
| Barrier film | | Type | E | | E | |
| | | Thickness (μm) | 22 | | 22 | |
| | | Length × width (mm) | 75 × 48 | | 75 × 24 | |
| | | Surface area (%) of portion where barrier film is disposed in 100% of total surface area of outer surface of container main body | 72 | | 36 | |
| | | Number of turns (circumference) of barrier film on outer circumferential surface of container main body | 1 | | 0.5 | |
| Liquid | | Type | ACD-A solution | | ACD-A solution | |
| Packaging material | | | - | | - | |
| Water vapor transmission rate (g/(m² ▪ day)) at 40°C and 0% RH of specimen collection | | | container 0.732 | | 0.947 | |
| Air transmission rate (cc/(m² ▪ day ▪ atm)) at 40°C and 0% RH of specimen collection container | | | 0.215 | | 0.490 | |
| | | | Immediately after manufacturing | After storage | Immediately after manufacturing | After storage |
| Amount of liquid contained (mL) | | | 1.5 | 1.33 | 1.5 | 1.23 |
| Amount of specimen collected (g) | | | 8.50 | 8.16 | 8.50 | 7.65 |
| Mixing ratio of liquid to specimen (amount of specimen/ amount of liquid) | | | 5.67 | 6.14 | 5.67 | 6.22 |
| Change rate (%) of mixing ratio | | | 8.3% | | 9.8% | |

[Table 7]

| | | Comparative Example 1 | | Comparative Example 2 | | Comparative Example 3 | | Comparative Example 4 | |
|---|---|---|---|---|---|---|---|---|---|
| Container main body | Type | A | | A | | A | | A | |
| | Material | PET | | PET | | PET | | PET | |
| | Length × outer diameter (mm) | 75 × 13 | | 75 × 13 | | 75 × 13 | | 75 × 13 | |
| Barrier film | Type | - | | - | | B | | C | |
| | Thickness (μm) | - | | - | | 50 | | 37 | |
| | Length × width (mm) | - | | - | | 50 × 40 | | 50 × 40 | |
| | Surface area (%) of portion where barrier film is disposed in 100% of total surface area of outer surface of container main body | - | | - | | 65 | | 65 | |
| | Number of turns (circumference) of barrier film on outer circumferential surface of container main body | - | | - | | 1 | | 1 | |
| Liquid | Type | Aqueous solution of citric acid | | Aqueous solution of citric acid | | Aqueous solution of citric acid | | Aqueous solution of citric acid | |
| Packaging material | | - | | Contained in aluminum gusset bag together with filter paper and deoxidizing agent | | - | | - | |
| Water vapor transmission rate (g/($m^2 \cdot$ day)) at 40°C and 0% RH of specimen collection container | | 1.193 | | 0.370 | | 1.100 | | 1.180 | |
| Air transmission rate (cc/($m^2 \cdot$ day $\cdot$ atm)) at 40°C and 0% RH of specimen collection container | | 0.524 | | -0.286 | | 0.510 | | 0.500 | |
| | | Immediately after manufacturing | After storage | Immediately after manufacturing | After storage | Immediately after manufacturing | After storage | Immediately after manufacturing | After storage |
| Amount of liquid contained (mL) | | 0.2 | 0.14 | 0.2 | 0.19 | 0.2 | 0.15 | 0.2 | 0.14 |
| Amount of specimen collected (g) | | 1.80 | 1.60 | 1.80 | 1.93 | 1.80 | 1.62 | 1.80 | 1.62 |

(continued)

| | Comparative Example 1 | | Comparative Example 2 | | Comparative Example 3 | | Comparative Example 4 | |
|---|---|---|---|---|---|---|---|---|
| Mixing ratio of liquid to specimen (amount of specimen/amount of liquid) | 9.00 | 11.44 | 9.00 | 10.14 | 9.00 | 10.95 | 9.00 | 11.25 |
| Change rate (%) of mixing ratio | 27.1% | | 12.6% | | 21.6% | | 25.0% | |

**EXPLANATION OF SYMBOLS**

1: Container main body
1a: Outer surface
2: Plug
3, 3A: Barrier film
4: Liquid
11, 11A: Specimen collection container
31: First film
32: Second film
33: First adhesive layer
34: Second adhesive layer

**Claims**

1. A specimen collection container into which a specimen is collected, the specimen collection container comprising:

   a container main body having an opening;
   a plug attached to the opening;
   a barrier film disposed on an outer surface of the container main body; and
   a liquid contained in the container main body,
   the barrier film having a water vapor transmission rate at 40°C and 90% RH of 0.8 g/(m$^2$ ▪ day) or less.

2. The specimen collection container according to claim 1, wherein an air transmission rate of the specimen collection container at 40°C and 0% RH is 0.5 cc/(m$^2$ ▪ day ▪ atm) or less.

3. The specimen collection container according to claim 1 or 2, wherein a water vapor transmission rate of the specimen collection container at 40°C and 0% RH is 1.1 g/(m$^2$ ▪ day) or less.

4. The specimen collection container according to any one of claims 1 to 3, wherein the barrier film is disposed at 0.2 turns or more and 3 turns or less in a circumferential direction of the outer surface of the container main body.

5. The specimen collection container according to any one of claims 1 to 4, wherein a surface area of a portion where the barrier film is disposed is 15% or more and 90% or less in 100% of a total surface area of the outer surface of the container main body.

6. The specimen collection container according to any one of claims 1 to 5, wherein a thickness of the barrier film before being disposed on the outer surface of the container main body is 5 um or more and 300 um or less.

7. The specimen collection container according to any one of claims 1 to 6, wherein

   the barrier film includes a first film, a first adhesive layer, a second film, and a second adhesive layer in this order, and
   the second adhesive layer is disposed on the outer surface of the container main body.

8. The specimen collection container according to claim 7, wherein

   the first film is a barrier film main body, and
   the second film is a polyethylene terephthalate film.

9. The specimen collection container according to any one of claims 1 to 8, wherein a material of the container main body is polyethylene terephthalate or polyethylene naphthalate.

10. The specimen collection container according to any one of claims 1 to 9, wherein

the specimen is blood, and
the liquid is a liquid including an anticoagulant.

11. The specimen collection container according to claim 10, wherein the anticoagulant is citric acid, EDTA, or heparin.

12. The specimen collection container according to any one of claims 1 to 11, wherein an amount of the liquid contained in the container main body is 0.1 mL or more and 5 mL or less.

[FIG. 1.]

11

2

1

1a

3a

34

32

3

33

31

4

[FIG. 2.]

11A

2

1

1a

3A

4

**EP 4 129 844 A1**

## INTERNATIONAL SEARCH REPORT

International application No.

PCT/JP2021/011919

**A. CLASSIFICATION OF SUBJECT MATTER**
Int.Cl. B65D23/08(2006.01)i, A61J3/00(2006.01)i, G01N1/10(2006.01)i, G01N33/48(2006.01)i
FI: G01N1/10N, G01N1/10V, G01N33/48K, A61J3/00300A, B65D23/08B
According to International Patent Classification (IPC) or to both national classification and IPC

**B. FIELDS SEARCHED**

Minimum documentation searched (classification system followed by classification symbols)
Int.Cl. B65D23/08, A61J3/00, G01N1/10, G01N33/48

Documentation searched other than minimum documentation to the extent that such documents are included in the fields searched
Published examined utility model applications of Japan      1922–1996
Published unexamined utility model applications of Japan    1971–2021
Registered utility model specifications of Japan            1996–2021
Published registered utility model applications of Japan    1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 2010/0254859 A1 (VACUTEST KIMA S.R.L.) 07 October 2010 (2010-10-07), paragraphs [0034]–[0069], fig. 1 | 1–12 |
| Y | JP 2020-29095 A (DAI NIPPON PRINTING CO., LTD.) 27 February 2020 (2020-02-27), paragraphs [0002], [0030], [0032], [0044] | 1–12 |
| Y | JP 9-253077 A (BECTON, DICKINSON AND COMPANY) 30 September 1997 (1997-09-30), paragraphs [0040], [0041], fig. 3 | 7–12 |
| A | US 2009/0162587 A1 (BECTON, DICKINSON AND COMPANY) 25 June 2009 (2009-06-25) | 1–12 |
| A | JP 2016-153785 A (INMAT INC.) 25 August 2016 (2016-08-25) | 1–12 |

☐  Further documents are listed in the continuation of Box C.      ☒  See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 20 May 2021 | 01 June 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

Information on patent family members

International application No.

PCT/JP2021/011919

| | | |
|---|---|---|
| US 2010/0254859 A1 | 07 October 2010 | WO 2007/072144 A2 |
| JP 2020-29095 A | 27 February 2020 | (Family: none) |
| JP 9-253077 A | 30 September 1997 | US 6165566 A<br>column 5, lines 24-42, fig. 3<br>EP 787826 A1 |
| US 2009/0162587 A1 | 25 June 2009 | WO 2009/085730 A1<br>CN 101932386 A |
| JP 2016-153785 A | 25 August 2016 | US 2009/0285722 A1<br>WO 2009/117129 A2<br>CN 102007049 A |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010154909 A